(19) Europäisches Patentamt
European Patent Office
Office européen des brevets

(11) EP 4 699 597 A1

(12) **EUROPEAN PATENT APPLICATION**
published in accordance with Art. 153(4) EPC

(43) Date of publication:
**25.02.2026 Bulletin 2026/09**

(21) Application number: **24792469.9**

(22) Date of filing: **29.03.2024**

(51) International Patent Classification (IPC):
***A61K 6/831*** *(2020.01)* ***A61K 6/20*** *(2020.01)*
***A61K 6/30*** *(2020.01)* ***A61K 6/40*** *(2020.01)*
***A61K 6/60*** *(2020.01)* ***A61K 6/71*** *(2020.01)*
***A61K 6/887*** *(2020.01)*

(52) Cooperative Patent Classification (CPC):
**A61K 6/20; A61K 6/30; A61K 6/40; A61K 6/60; A61K 6/71; A61K 6/831; A61K 6/887**

(86) International application number:
**PCT/JP2024/013054**

(87) International publication number:
**WO 2024/219193 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.04.2023 JP 2023068578**

(71) Applicant: **GC R&D Corporation**
**Tokyo 174-8585 (JP)**

(72) Inventors:
- **TANAZAWA, Kimitaka**
  **Tokyo 174-8585 (JP)**
- **SHOJI, Takumi**
  **Tokyo 174-8585 (JP)**
- **USUKI, Daisuke**
  **Tokyo 174-8585 (JP)**
- **HIGUCHI, Koji**
  **Tokyo 174-8585 (JP)**

(74) Representative: **Müller-Boré & Partner Patentanwälte PartG mbB**
**Friedenheimer Brücke 21**
**80639 München (DE)**

# (54) DENTAL COMPOSITION, RESIN MATERIAL FOR DENTAL CUTTING, AND METHOD FOR PRODUCING RESIN MATERIAL FOR DENTAL CUTTING

(57) A dental composition includes a polymerizable monomer including only one group selected from the group consisting of a urethane group, a urea group, and a carbonate group; and an inorganic filler.



Processed by Luminess, 75001 PARIS (FR)

## Description

### TECHNICAL FIELD

The present invention relates to dental compositions, dental milling resin materials, and methods of producing dental milling resin materials.

### BACKGROUND ART

In recent years, prostheses, such as crowns, bridges, or the like, are designed on-screen using computers, and CAD/CAM devices that perform milling to produce prostheses are used in production of dental prostheses. Thus, dental prostheses with consistent quality can be reliably supplied in a short time.

For milling performed by the CAD/CAM devices, a dental milling resin material (also referred to as a dental resin block or dental milling blank), which is obtained by forming a dental resin into a block, is used as a material before cutting into a shape (see, for example, Patent Document 1).

### CITATION LIST

### PATENT DOCUMENT

Patent Document 1: Japanese Patent No. 6739809

### SUMMARY OF THE INVENTION

### TECHNICAL PROBLEM

As the above-described dental milling resin material, a resin material having a higher mechanical strength is desired in order to ensure stability of a prosthesis in the oral cavity.

An object of the present invention is to provide a dental composition with which a dental milling resin material having a high mechanical strength can be obtained.

### SOLUTION TO THE PROBLEM

According to one aspect of the present invention, a dental composition includes a polymerizable monomer including only one group selected from the group consisting of a urethane group, a urea group, and a carbonate group; and an inorganic filler.

### EFFECTS OF THE INVENTION

According to one aspect of the present invention, a dental composition, with which a dental milling resin material having a mechanical strength can be obtained, is provided.

### DETAILED DESCRIPTION OF THE EMBODIMENTS

Next, an embodiment for implementing the present invention will be described.

<Dental composition>

The dental composition of the present embodiment includes a polymerizable monomer including only one group selected from the group consisting of a urethane group, a urea group, and a carbonate group (referred to as one urethane group or the like hereinafter); and an inorganic filler. In the present specification, the dental composition refers to a composition used in the field of dentistry.

The urethane group is a bonding group represented by -NHCOO-. The urea group is a bonding group represented by -NHCONH-. The carbonate group is a bonding group represented by -OCOO-.

The polymerizable monomer including only one urethane group or the like includes one urethane group or the like, but does not include two or more urethane groups or the like.

The polymerizable monomer including only one urethane group or the like is not particularly limited. For example, the polymerizable monomer including only one urethane group or the like is (meth)acrylate including one urethane group

or the like. In the present specification, the (meth)acrylate refers to a compound including a methacryloyloxy group, an acryloyloxy group, or both (referred to as a (meth)acryloyloxy group hereinafter).

The (meth)acrylate including only one urethane group or the like is preferably (meth)acrylate including two or more (meth)acryloyloxy groups.

The (meth)acrylate including only one urethane group or the like is, for example, represented by the following formula (1).

[Chem. 1]

$$A_1 - R_1 - B - R_2 - A_2 \qquad (1)$$

In the formula (1), $A_1$ and $A_2$ are each independently a (meth)acryloyloxy group or (meth)acrylamide group that may include an alkyl group having a carbon number of 3 or less in a side chain; B is a urethane group; and $R_1$ and $R_2$ are each independently an alkyl group having a carbon number of 8 or less, in which a (meth)acryloyloxy group or a (meth) acrylamide group may be included in a side chain.

The (meth)acrylamide group refers to a methacrylamide group, an acrylamide group, or both. The acrylamide group refers to a substituent represented by $-NHCO(CH=CH_2)$.

In $A_1$ and $A_2$, it is preferable that the carbon number of the alkyl group included in the side chain of the (meth) acryloyloxy group or the (meth)acrylamide group is 2 or less, or the side chain of the (meth)acryloyloxy group or the (meth) acrylamide group does not include an alkyl group. In addition, in $R_1$ and $R_2$, the carbon number of the alkyl group is preferably 5 or less.

When the carbon number of the alkyl group is 9 or greater in $R_1$ and $R_2$, the polymerizable monomer including only one urethane group or the like is easily distorted, and a cured product obtained by curing a dental composition including such a polymerizable monomer cannot have a sufficient mechanical strength.

The (meth)acrylate represented by the above formula (1) is not particularly limited. For example, (meth)acrylate represented by the above formula (1) is (meth)acrylate including only one urethane group (-NHCOO-) represented by any of the following formulae (2) to (6).

[Chem. 2]

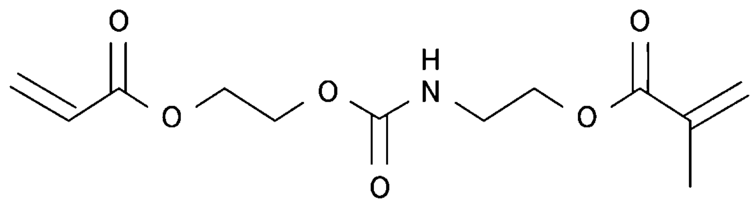

(2)

[Chem. 3]

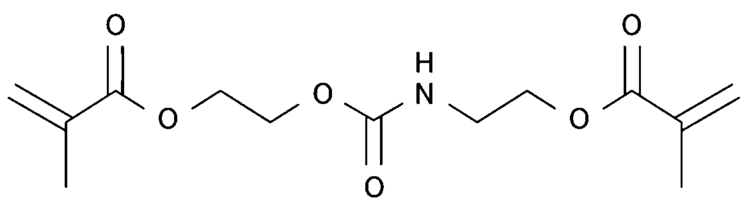

(3)

[Chem. 4]

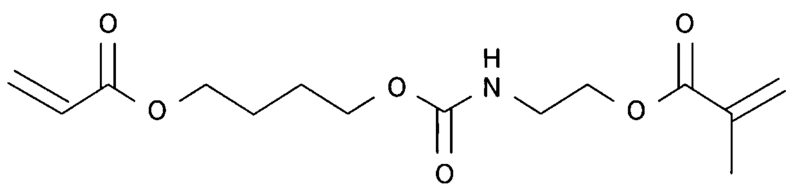

(4)

[Chem. 5]

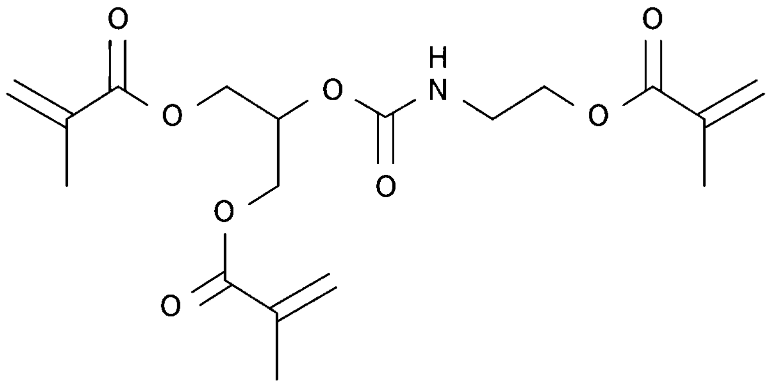

(5)

[Chem. 6]

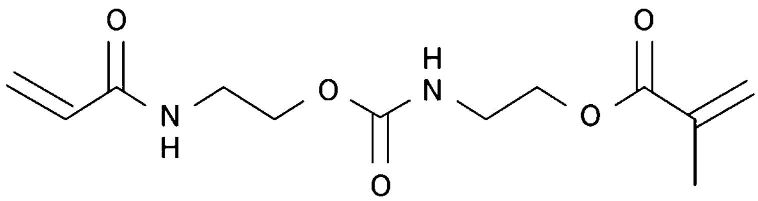

(6)

An amount of the polymerizable monomer including only one urethane group or the like in the dental composition is not particularly limited, but is preferably 5 percent by mass or greater and 30 percent by mass or less, more preferably 10 percent by mass or greater and 28 percent by mass or less, and yet more preferably 15 percent by mass or greater and 25 percent by mass or less. When the amount of the polymerizable monomer including only one urethane group or the like is 5 percent by mass or greater and 30 percent by mass or less, a cured product of the dental composition can maintain a high mechanical strength.

The amount of the polymerizable monomer including only one urethane group or the like varies depending on the intended use of the dental composition. In the case where the intended use of the dental composition is a dental milling resin material, the amount of the polymerizable monomer including only one urethane group or the like is preferably 5 percent by mass or the like and 30 percent by mass or less, and more preferably 10 percent by mass or the like and 25 percent by mass or less.

The inorganic filler is formed of an inorganic material, and the inorganic filler is included in a resin composition in a form of particles. The inorganic filler is not particularly limited. Examples of the inorganic filler include barium glass, strontium glass a silica powder, and the like.

As the inorganic filler, an inorganic filler subjected to a hydrophobic treatment is preferably used. For the hydrophobic treatment of the inorganic filler, for example, a silane-coupling agent is used. Examples of the silane-coupling agent include 3-methacryloyloxypropyltrimethoxysilane, 8-methacryloyloxyoctyltrimethoxysilane, and the like.

A usage amount of the silane-coupling agent is preferably 0.1 percent by mass or greater and 15 percent by mass or less, more preferably 0.5 percent by mass or greater and 10 percent by mass or less, and yet more preferably 1 percent by mass or greater and 5 percent by mass or less, relative to 100 percent by mass of the inorganic material constituting the inorganic filler.

A particle size of the main inorganic filler is, for example, preferably 0.1 μm or greater and 100 μm or less, more preferably 0.1 μm or greater and 70 μm or less, and yet more preferably 0.1 μm or greater and 50 μm or less. The particle size refers to an average particle diameter defined by a median diameter (d50).

An amount of the inorganic filler in the dental composition is not particularly limited, but is preferably 70 percent by mass or greater and 90 percent by mass or less, more preferably 72 percent by mass or greater and 85 percent by mass or less, and yet more preferably 75 percent by mass or greater and 82 percent by mass or less. When the amount of the inorganic filler is less than 70 percent by mass, a sufficient mechanical strength cannot be obtained. When the amount of the inorganic filler is greater than 90 percent by mass, a viscosity of the dental composition is significantly increased, and therefore a problem may be caused, such that air bubbles are included in a cured product at the time when the dental composition is cured.

An amount of the inorganic filler in the dental composition varies depending on the intended use of the dental

composition. In the case where the intended use of the dental composition is a dental milling resin material, the amount of the inorganic filler is preferably 70 percent by mass or greater and 90 percent by mass or less, and more preferably 75 percent by mass or greater and 85 percent by mass or less.

The dental composition of the present embodiment may further include polymerizable monomers other than the polymerizable monomer including only one urethane group or the like (referred to as other polymerizable monomers hereinafter).

As other polymerizable monomers, (meth)acrylate that does not include a urethane group, and (meth)acrylate including two or more urethane groups can be used.

Examples of other polymerizable monomers include 2,2-bis(4-methacryloyloxypolyethoxyphenyl)propane (Bis-MPEPP), glycerol dimethacrylate (GDMA), neopentyl glycol dimethacrylate (NPGDMA), triethylene glycol dimethacrylate (TEGDMA), bisphenol A glycidyl methacrylate (Bis-GMA), tricyclodecane dimethanol dimethacrylate (DCP), trimethylolpropane trimethacrylate (TMPT), dipentaerythritol hexaacrylate (A-DPH), and the like.

Examples of the (meth)acrylate including two or more urethane groups include di-2-methacryloyloxyethyl-2,2,4-trimethylhexamethylene dicarbamate (UDMA), and the like.

As other polymerizable monomers, one polymerizable monomer may be used alone, or two or more polymerizable monomers may be used in combination.

An amount of other polymerizable monomers in the dental composition is not particularly limited, but is preferably 1 percent by mass or greater and 70 percent by mass or less, more preferably 5 percent by mass or greater and 60 percent by mass or less, and yet more preferably 10 percent by mass or greater and 50 percent by mass or less. When the amount of other polymerizable monomers is 1 percent by mass or greater and 70 percent by mass or less, a cured product of the dental composition can maintain a high mechanical strength.

The dental composition of the present embodiment may optionally include other ingredients as long as other ingredients do not adversely affect the object of the present invention. Examples of other ingredients included in the dental composition include a polymerization initiator, a polymerization accelerator, a polymerization inhibitor, a chain transfer agent, a filler (excluding the above inorganic filler), a coloring material, a pigment, a fluorescent agent, an antibacterial agent, and the like.

Examples of the polymerization initiator include organic peroxide, such as benzoyl peroxide and the like. As the polymerization initiator, one polymerization initiator may be used alone, or two or more polymerization initiators may be used in combination.

An amount of the polymerization initiator in the dental composition is not particularly limited, but is, for example, 0.005 percent by mass or greater and 10 mass or less, preferably 0.01 percent by mass or greater and 5 percent by mass or less, and yet more preferably 0.1 percent by mass or greater and 3 percent by mass or less. When the amount of the polymerization initiator in the dental composition is 0.005 percent by mass or greater and 10 mass or less, a polymerization efficiency at the time when the dental composition is cured is improved.

Examples of the filler include: inorganic fillers other than the above inorganic filler, such as silica zirconia, silica alumina, and the like; organic-inorganic hybrid fillers; cluster fillers; and the like. As the filler, a filler subjected to a hydrophobic treatment is preferably used. For the hydrophobic treatment of the filler, for example, a silane-coupling agent, such as 3-methacryloyloxypropyltrimethoxysilane or the like, can be used.

Examples of the pigment include iron oxide, titanium oxide, and the like.

Examples of the fluorescent agent include diethyl-2,5-dihydroxyterephthalate, and the like.

As described above, the dental composition of the present embodiment includes the polymerizable monomer including only one urethane group or the like. The polymerizable monomer including only one urethane group or the like tends to have a relatively low viscosity compared to a polymerizable monomer including two or more urethane groups. Therefore, as the dental composition includes the polymerizable monomer including only one urethane group or the like, an amount of the inorganic filler in the dental composition can be increased compared to a dental composition of a related art so that a mechanical strength (e.g., flexural strength) of a polymerization cured product of the dental composition can be improved.

The dental composition of the present embodiment can be used for various dental materials because an effect of improving a mechanical strength of a polymerization cured product can be obtained as described above.

Examples of the dental materials include dental milling resin materials, composite resins for dental filling, dental hard resins, dental temporary restorative materials, dental fillers, dental cements, dental adhesives (including primers for crown restoration adhesion), dental primers, tooth surface coating materials, dentifrices, and the like. Among the above dental materials, the dental composition of the present embodiment is suitably used for a dental milling resin material.

<Dental milling resin material>

The dental milling resin material of the present embodiment can be formed by curing the above-described dental composition through polymerization. Specifically, the above-described dental composition is used in the dental milling

resin material of the present embodiment.

An embodiment of curing the dental composition through polymerization is not particularly limited. Examples of the embodiment include polymerization methods, such as thermal polymerization, chemical polymerization, photopolymerization, and the like.

Since the dental composition of the present embodiment is used in the dental milling resin material of the present embodiment, the effects of the dental composition can be obtained as they are. Specifically, a mechanical strength of the dental milling resin material obtained as a cured product can be improved by curing the dental composition, which includes the polymerizable monomer including only one urethane group or the like, and the inorganic filler, through polymerization.

<Method of producing dental milling resin material>

The method of producing the dental milling resin material according to the present embodiment includes curing the above-described dental composition through polymerization. Specifically, the above-described dental composition is used in the method of producing the dental milling resin material according to the present embodiment.

An embodiment of curing the dental composition through polymerization is not particularly limited, and the same polymerization method (e.g., thermal polymerization, chemical polymerization, photopolymerization, or the like) as the polymerization method used for the above-described dental milling resin material when the dental composition is polymerized and cured is used.

The polymerization conditions for curing the dental composition through polymerization are arbitrary. For example, the polymerization temperature is 50°C or higher and 200°C or lower, preferably 80°C or higher and 180°C or lower, and more preferably 110°C or higher and 150°C or lower. In addition, the duration of the polymerization is 20 minutes or longer and 360 minutes or shorter, preferably 60 minutes or longer and 240 minutes or shorter, and more preferably 100 minutes or longer and 180 minutes or shorter.

The polymerization of the dental composition may be performed under pressure. In this case, the pressure is 1.0 MPa or greater and 10.0 MPa or less, preferably 2.0 MPa or greater and 8.0 MPa or less, and more preferably 3.0 MPa or greater and 5.0 MPa or less.

Since the dental composition of the present embodiment is used in the method of producing the dental milling resin material according to the present embodiment, the effects of the dental composition are obtained as they are. Specifically, a mechanical strength of the dental milling resin material obtained as a cured product can be improved by curing the dental composition, which includes the polymerizable monomer including only one urethane group or the like, and the inorganic filler, through polymerization.

In the method of producing the dental milling resin material according to the present embodiment, a dental milling resin material can be produced merely by using the polymerizable monomer including only one urethane group or the like as a polymerizable monomer to be included in a dental composition. Specifically, a dental milling resin material can be produced using only a single monomer in the production method of the present embodiment.

In the case where monomers are mixed and blended, instead of use of a single monomer, there is a risk such that a variation in quality is concerned depending on the purity of other monomers (the risk increases the number of monomer increases). In addition, in the case where monomers are mixed and blended, instead of use of a single monomer, monomers may not be homogeneously mixed (the risk of decreasing the homogeneousness of the quality increases).

Conversely, a dental milling resin material can be producing using only a single monomer in the method of producing the dental milling resin material according to the present embodiment, and therefore a risk of contamination with foreign matter can be reduced because of reduction in the number of production steps. In addition, since the dental milling resin material is produced using only a single monomer, the production process can be simplified so that the production cost can be reduced in terms of materials, facilities, or the like.

Examples

Examples of the present invention will be described hereinafter, but the present invention is not limited to Examples. Numerical values without units are based on mass (percent by mass), unless otherwise specified.

<Production of dental milling resin material (resin block)>

As a dental composition, a dental milling resin material (resin block) was produced. First, a curable polymerizable monomer (monomer) and a polymerization initiator (BPO) were mixed at a composition ratio presented in Tables 1 and 2 to prepare a monomer liquid.

The ingredients presented by symbols or abbreviations in Tables 1 and 2 are as follows. mUDMA: (meth)acrylate represented by the above formula (3)

mUA/MA-1: (meth)acrylate represented by the above formula (2)
mUA/MA-2: (meth)acrylate represented by the above formula (4)
mUTMA: (meth)acrylate represented by the above formula (5)
mUAA/MA: (meth)acrylate represented by the above formula (6)
UDMA: di-2-methacryloyloxyethyl-2,2,4-trimethylhexamethylenedicarbamate
D-2.6E: 2,2-bis(4-methacryloyloxy
polyethoxyphenyl)propane, in which the average number of moles of the ethoxy group added is 2.6
GDMA: glycerol dimethacrylate
NPGDMA: neopentyl glycol dimethacrylate
TEGDMA: triethylene glycol dimethacrylate
BPO: benzoyl peroxide (polymerization initiator)

The monomer liquid and the inorganic filler (silica powder + silane-treated glass) were mixed at a blending ratio [percent by mass] presented in Tables 3 and 4 by a planetary centrifugal mixer (or a double planetary mixer) to prepare a paste. A molding container was filled with the resultant paste, and the paste was then polymerized and cured in a nitrogen gas at the pressure of 1.0 MPa to 5.0 MPa for 60 minutes to 180 minutes at 100°C to 130°C to obtain a cured block (Examples 1 to 16 and Comparative Examples 1 to 5).

The ingredients presented by symbols or abbreviations in Tables 3 and 4 are as follows. Silica powder: AEROSIL (manufactured by NIPPON AEROSIL CO., LTD., AEROSIL is a registered trademark) Silane-treated glass: a barium glass powder surface-treated with 8-methacryloyloxyoctyltrimethoxysilane (average particle diameter: 0.7 μm)

<Paste viscosity>

The viscosity of the paste was measured at 25°C at the shear rate of 1 [1/s] by a rheometer (MCR 302e, manufactured by Anton Paar Japan K.K.).

<Flexural strength test>

A specimen in the size of 1.2 mm × 4.0 mm × 14.0 mm was cut out from the cured block by a diamond cutter, and each surface of the specimen was finished with water-resistant abrasive paper of P2000. A three-point flexural strength test was performed by a universal testing machine (AG-Xplus, manufactured by Shimadzu Corporation) under the conditions such that a distance between supporting points was 12 mm and a cross head speed was 1.0 mm/min, to measure a three-point flexural strength and flexural modulus.

The three-point flexural strength was calculated according to the following equation (7).

[Math. 1]

$$[MP_a] = \quad 3FS/(2bh^2) \qquad (7)$$

In the equation (7), F is the maximum load [N] measured in the flexural strength test, S is the distance [mm] between supporting points, b is a width [mm] of the specimen measured immediately before the test, and h is a thickness [mm] of the specimen measured immediately before the test. As the evaluation of the three-point flexural strength, the result was determined as good when the three-point flexural strength was 310 MPa or greater, and the result was determined as not good when the three-point flexural strength was less than 310 MPa.

The flexural modulus was calculated from a strain-stress curve obtained in the three-point flexural strength test according to the secant method. As the evaluation of the flexural modulus, the result was determined as good when the flexural modulus was 8 GPa or greater, and the result was determined as not good when the flexural modulus was less than 8 GPa.

<Milling processability>

Contact processability when the cured block was milled by a milling machine (Aadva Harmony WET, manufactured by GC Corporation) was examined. The contact processability was evaluated based on whether the contact processability of the cured block was comparable to contact processability of a dental milling resin material of a related art (cured block of a related art).

[Table 1]

| Monomer liquid composition | Example | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| mUDMA | 100 | 100 | 100 | 100 | 100 | 100 | | | 80 | 60 | 40 | | | | 80 | 60 |
| mUA/MA-1 | | | | | | | 100 | 100 | | | | | | | | |
| mUA/MA-2 | | | | | | | | | | | | 100 | | | | |
| mUTMA | | | | | | | | | | | | | 100 | 100 | | |
| mUAA/MA | | | | | | | | | | | | | | | 20 | 40 |
| UDMA | | | | | | | | | 10 | 20 | 30 | | | | | |
| D-2.6E | | | | | | | | | | | | | | | | |
| GDMA | | | | | | | | | 10 | 10 | 20 | | | | | |
| NPGDMA | | | | | | | | | | 10 | 10 | | | | | |
| TEGDMA | | | | | | | | | | | | | | | | |
| BPO | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| Total | 101.5 | 101.5 | 101.5 | 101.5 | 101.5 | 101.5 | 101.5 | 101.5 | 101.5 | 101.5 | 101.5 | 101.5 | 101.5 | 101.5 | 101.5 | 101.5 |

[Table 2]

| Monomer liquid composition | Comparative Example | | | | |
|---|---|---|---|---|---|
| | 1 | 2 | 3 | 4 | 5 |
| mUDMA | | | | | |
| mUA/MA-1 | | | | | |
| mUA/MA-2 | | | | | |
| mUTMA | | | | | |
| mUAA/MA | | | | | |
| UDMA | 100 | | 60 | 85 | 60 |
| D-2.6E | | 100 | 30 | | 30 |
| GDMA | | | 10 | 10 | |
| NPGDMA | | | | 5 | |
| TEGDMA | | | | | 10 |
| BPO | 1.5 | 1.5 | 1.5 | 1.5 | 1.5 |
| Total | 101.5 | 101.5 | 101.5 | 101.5 | 101.5 |

[Table 3]

| | Example | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| Monomer liquid | 22 | 21 | 20 | 19.5 | 19 | 18 | 22 | 20 | 20 | 20 | 20 | 20 | 22 | 20 | 20 | 20 |
| Inorganic filler (silica powder + silane-treated glass) | 78 | 79 | 80 | 80.5 | 81 | 82 | 78 | 80 | 80 | 80 | 80 | 80 | 78 | 80 | 80 | 80 |
| Total | 100 | 100 | 100 | 100 | 100 | 100 | 100 | 100 | 100 | 100 | 100 | 100 | 100 | 100 | 100 | 100 |
| Paste viscosity [Pa·s] | 14.1 | 17.8 | 24.3 | 28.7 | 37.0 | 68.0 | 16.3 | 29.8 | 26.8 | 23.7 | 28.2 | 21.1 | 47.5 | 135 | 29.1 | 34.5 |
| 3-point flexural strength [MPa] | 394 | 369 | 384 | 382 | 386 | 363 | 365 | 371 | 347 | 350 | 362 | 319 | 312 | 313 | 384 | 405 |
| Flexural modulus [GPa] | 12.4 | 12.6 | 12.9 | 13.6 | 13.2 | 13.2 | 12.5 | 12.6 | 12.3 | 12.7 | 12.4 | 10.9 | 12.7 | 13.2 | 13.2 | 13.2 |
| Milling processability | Good | Good | Good | Good | Good | Good | Good | Good | Good | Good | Good | Good | Good | Good | Good | Good |

[Table 4]

| | Comparative Example | | | | |
|---|---|---|---|---|---|
| | 1 | 2 | 3 | 4 | 5 |
| Monomer liquid | 29.4 | 29.4 | 22.8 | 20.5 | 21.2 |
| Inorganic filler (silica powder + silane-treated glass | 70.6 | 70.6 | 77.2 | 79.5 | 78.8 |
| Total | 100 | 100 | 100 | 100 | 100 |
| Paste viscosity [Pa·s] | 123 | 162 | 201 | 255 | 194 |
| 3-point flexural strength [MPa] | 244 | 219 | 305 | 307 | 296 |
| Flexural modulus [GPa] | 7.7 | 7.1 | 10.7 | 11.8 | 11.0 |
| Milling processability | Good | Good | Good | Good | Good |

With reference to Tables 1 and 3, in Examples 1 to 16, the resin block obtained using the dental composition including the polymerizable monomer including only one urethane group and the inorganic filler had the desired results in both the three-point flexural strength and the flexural modulus.

Conversely, with reference to Tables 2 and 4, in Comparative Examples 1 to 5, the resin block obtained using the dental composition that did not include the polymerizable monomer including only one urethane group had the undesirable result in the three-point flexural strength. In addition, in Comparative Examples 1 to 2, the result of the flexural modulus was not good.

Although the embodiments of the present invention have been described above, the present invention is not limited to the specific embodiments, and various modifications and changes can be made within the scope of the invention described in the claims.

The present application is based on and claims priority to Japanese Patent Application No. 2023-068578, filed on April 19, 2023, the entire contents of which are incorporated herein by reference.

## Claims

**1.** A dental composition comprising:

a polymerizable monomer including only one group selected from the group consisting of a urethane group, a urea group, and a carbonate group; and
an inorganic filler.

**2.** A dental milling resin material, obtained by curing the dental composition according to claim 1 through polymerization.

**3.** A method of producing a dental milling resin material, the method comprising:
curing the dental composition according to claim 1 through polymerization.

## INTERNATIONAL SEARCH REPORT

International application No.
**PCT/JP2024/013054**

**A. CLASSIFICATION OF SUBJECT MATTER**

***A61K 6/831***(2020.01)i; ***A61K 6/20***(2020.01)i; ***A61K 6/30***(2020.01)i; ***A61K 6/40***(2020.01)i; ***A61K 6/60***(2020.01)i; ***A61K 6/71***(2020.01)i; ***A61K 6/887***(2020.01)i
FI: A61K6/831; A61K6/20; A61K6/30; A61K6/40; A61K6/60; A61K6/71; A61K6/887

According to International Patent Classification (IPC) or to both national classification and IPC

**B. FIELDS SEARCHED**

Minimum documentation searched (classification system followed by classification symbols)

A61K6/831; A61K6/20; A61K6/30; A61K6/40; A61K6/60; A61K6/71; A61K6/887

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2021-138673 A (TOKUYAMA DENTAL CORP.) 16 September 2021 (2021-09-16) claims, paragraphs [0001]-[0003], examples | 1-3 |
| X | JP 2004-300066 A (MITSUI CHEMICALS, INC.) 28 October 2004 (2004-10-28) claims, paragraphs [0006], [0012], [0014], [0073], examples | 1-3 |
| X | JP 2023-510962 A (IVOCLAR VIVADENT AG) 15 March 2023 (2023-03-15) claims, paragraphs [0033], [0050]-[0061], [0116]-[0119], examples | 1-3 |
| X | JP 63-107954 A (TOAGOSEI CO., LTD.) 12 May 1988 (1988-05-12) claims, page 2, upper left column, line 18 to upper right column, line 2, page 3, lower right column, line 16 to page 4, upper left column, line 1, examples | 1-3 |
| X<br>A | JP 2014-091692 A (KURARAY NORITAKE DENTAL INC.) 19 May 2014 (2014-05-19) claims, examples | 1<br>2, 3 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 May 2024** | **11 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/013054**

**C. DOCUMENTS CONSIDERED TO BE RELEVANT**

| Category* | Citation of document, with indication, where appropriate, of the relevant passages | Relevant to claim No. |
|---|---|---|
| A | JP 8-034707 A (TOAGOSEI CO., LTD.) 06 February 1996 (1996-02-06) entire text, all drawings | 1-3 |
| A | JP 8-188630 A (DAICEL CHEM. IND. LTD.) 23 July 1996 (1996-07-23) entire text, all drawings | 1-3 |
| A | JP 2017-025058 A (IVOCLAR VIVADENT AG) 02 February 2017 (2017-02-02) entire text, all drawings | 1-3 |
| A | JP 2015-508819 A (DENTSPLY INTERNATIONAL INC.) 23 March 2015 (2015-03-23) entire text, all drawings | 1-3 |
| A | LU, Hui et al., Development of highly reactive mono-(meth)acrylates as reactive diluents for dimethacrylate-based dental resin systems, Biomaterials, 2005, 26, 1329-1336, DOI:10.1016/j.biomaterials.2004.04.041 entire text, all drawings | 1-3 |
| A | ALTIN, Ayse et al., Synthesis and photopolymerization of novel, highly reactive phosphonated-urea-methacrylates for dental materials, Reactive and Functional Polymers, 2013, 73, 1319-1326, DOI:10.1016/j.reactfunctpolym.2013.07.006 entire text, all drawings | 1-3 |
| A | MOSZNER, Norbert et al., New Diluents for Dental Composites, Macromol. Mater. Eng., 2016, 301, 750-759, DOI:10.1002/mame.201600115 entire text, all drawings | 1-3 |

Form PCT/ISA/210 (second sheet) (July 2022)

**INTERNATIONAL SEARCH REPORT**
**Information on patent family members**

International application No.
**PCT/JP2024/013054**

| Patent document cited in search report | Publication date (day/month/year) | Patent family member(s) | Publication date (day/month/year) |
|---|---|---|---|
| JP 2021-138673 A | 16 September 2021 | (Family: none) | |
| JP 2004-300066 A | 28 October 2004 | (Family: none) | |
| JP 2023-510962 A | 15 March 2023 | US 2023/0059534 A1<br>claims, paragraphs [0044], [0161], examples<br>EP 3854374 A1<br>CN 114845683 A | |
| JP 63-107954 A | 12 May 1988 | (Family: none) | |
| JP 2014-091692 A | 19 May 2014 | (Family: none) | |
| JP 8-034707 A | 06 February 1996 | (Family: none) | |
| JP 8-188630 A | 23 July 1996 | (Family: none) | |
| JP 2017-025058 A | 02 February 2017 | US 2017/0020791 A1<br>entire text, all drawings<br>EP 3120827 A1 | |
| JP 2015-508819 A | 23 March 2015 | US 2013/0225716 A1<br>entire text, all drawings<br>EP 2782545 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6739809 B
- JP 2023068578 A